# EUROPÄISCHE PATENTANMELDUNG

(11) **EP 2 050 429 A1**
(43) Veröffentlichungstag der Anmeldung: **22.04.2009**
(21) Anmeldenummer: 08016920.4
(22) Anmeldetag: 25.09.2008
(51) Int. Cl.: A61H 23/04, A61F 5/01, A61F 13/06

(54) **Sprunggelenkschiene mit einem ein massierendes Fluidpolsterkissen aufweisenden Pronationsgurt**

(30) Priorität: 18.10.2007 DE 102007049953
(71) Anmelder: Albrecht GmbH, 83115 Neubeuern (DE)
(72) Erfinder: Albrecht, Erich, 83115 Neubeuern (DE)
(74) Vertreter: Bauer, Friedrich

(57) **Zusammenfassung**

Eine Sprunggelenkschiene weist einen Pronationsgurt (11) mit einem Sohlenabschnitt (17) und einem Lateral-/Ristabschnitt (18) auf, wobei in diesen Abschnitten des Pronationsgurtes (11) ein als durchgehendes Massagekissen ausgebildetes Luftpolsterkissen (12) vorgesehen ist. Das Luftpolsterkissen (12) ist derart ausgebildet, dass das im Luftpolsterkissen (12) enthaltene Fluid dynamisch vom Sohlenabschnitt (17) in den Latral-/Ristabschnitt (18) und umgekehrt gepumpt wird.

## Beschreibung

Die Erfindung betrifft eine Sprunggelenkschiene mit einem Pronationsgurt gemäß dem Oberbegriff des Patentanspruchs 1.

Sprunggelenkschienen (Sprunggelenkorthesen) in der Form von funktionellen U-Schienen haben sich bei der Rehabilitation von Verletzungen bzw. degenerativen Erkrankungen des Sprunggelenkes bewährt. Derartige Sprunggelenkschienen weisen relativ steife Seitenteile auf, die auf der medialen und lateralen Seite des Unterschenkels anlegbar und an diesem beispielsweise mittels Klettbändern befestigbar sind, die zirkulär um die Seitenteile herumgeführt werden. Die Seitenteile erstrecken sich dabei über das Sprunggelenk nach unten bis in einen sohlennahen Bereich des Fußes, wo sie mittels eines Querbandes, das unter der Ferse hindurchgeführt wird, miteinander verbunden sind. Derartige U-Schienen stützen das Sprunggelenk medial und lateral, d.h auf beiden Seiten, und lassen Flexion und Extension des Sprunggelenks weitestgehend zu, so dass ein normaler Bewegungsablauf erfolgen kann.

Bei bestimmten Verletzungen im Sprunggelenkbereich, insbesondere bei Verletzungen am Bandapparat des Sprunggelenks und bei bereits gelockertem Gelenk, kann es wünschenswert sein, zusätzlich zur Sprunggelenkschiene einen Pronationsgurt zu verwenden, der die Außenbänder des Sprunggelenks durch Anheben des Vorfußes entlastet und einer Supination des Fußes entgegenwirkt. Der Einsatz eines derartigen Pronationsgurtes ist auch bei konservativer oder funktioneller Nachbehandlung von Sprunggelenksfrakturen und Bandrupturen üblich. Üblicherweise erstreckt sich dabei der Pronationsgurt vom medialen Seitenteil der Sprunggelenkschiene unterhalb der Fußsohle nach außen und von dort über den Außenrand des Fußes über den Fußrücken (Rist) zurück zum medialen Seitenteil.

Ausgehend von einem derartigen Stand der Technik liegt der Erfindung die Aufgabe zugrunde, eine Sprunggelenkschiene mit einem Pronationsgurt der eingangs genannten Art zu schaffen, mit dem die Regeneration des Fußes, insbesondere im Bereich der Außenbänder, auf besonders wirkungsvolle Weise unterstützt werden kann.

Diese Aufgabe wird erfindungsgemäß durch eine Sprunggelenkschiene mit den Merkmalen des Anspruchs 1 gelöst. Vorteilhafte Ausführungsformen der Erfindung sind in den weiteren Ansprüchen beschrieben.

Bei der erfindungsgemäßen Sprunggelenkschiene weist der Sohlenabschnitt und Lateral-/Ristabschnitt des Pronationsgurtes ein in beiden Abschnitten angeordnetes, als durchgehendes Massagekissen ausgebildetes Fluidpolsterkissen mit Fluidfüllung auf, derart, dass zumindest ein Teil des im Fluidpolsterkissen enthaltenen Fluids bei Komprimierung des Sohlenabschnittes in den Lateral-/Ristabschnitt gepumpt und bei Entlastung des Sohlenabschnittes und Komprimierung des Lateral-/Ristabschnittes in den Sohlenabschnitt gepumpt wird, wodurch das Fluidpolsterkissen im Bereich der Fußsohle der lateralen Fußaußenseite und des Fußrückens eine dynamische Dickenänderung erfährt.

Mit Hilfe des erfindungsgemäßen Fluidpolsterkissens, das insbesondere als Luftpolsterkissen, jedoch auch als Flüssigkeitspolsterkissen ausgebildet sein kann, wird das im Fluidpolsterkissen enthaltene Fluid beim Gehen abwechselnd vom Sohlenabschnitt nach oben in den Lateral-/Ristabschnitt des Pronationsgurtes und von dort wieder nach unten in den Sohlenabschnitt gepumpt. Dies wird auf einfache Weise dadurch bewirkt, dass beim Aufsetzen des Fußes auf den Untergrund der Sohlenabschnitt komprimiert und beim Abheben des Fußes vom Untergrund wieder entlastet wird. Das hin- und herströmende Fluid bewirkt eine laufende, dynamische Druckänderung auf diejenigen Bereiche des Fußes, an denen das Fluidpolsterkissen anliegt. Dies führt zu einem intensiven Massageeffekt, der sich von der Fußsohle über den lateral Außenrand des Fußes bis in den Fußrücken hinein erstreckt, wodurch die Durchblutung in diesem Bereich intensiv angeregt und das Gewebe gelockert wird. Es kann somit insbesondere auch verhindert werden, dass es in diesen Bereichen des Fußes zu Blutstauungen kommt. Das massierende Fluidpolsterkissen hat damit sehr positive Auswirkungen auf die Regeneration von beschädigtem Gewebe. Weiterhin wirkt der Massageeffekt entspannend, wodurch auch der Tragekomfort des Pronationsgurtes erhöht wird.

Gemäß einer vorteilhaften Ausführungsform erstreckt sich das Fluidpolsterkissen über die laterale Sohlenhälfte des Mittelfußes und von dort bis zum oberen Ende des Fußrückens. Alternativ hierzu ist es jedoch auch denkbar, dass sich das Fluidpolsterkissen im Sohlenabschnitt weiter medial nach innen, beispielsweise bis zum medialen Rand der Fußsohle, erstreckt.

Gemäß einer vorteilhaften Ausführungsform besteht das Fluidpolsterkissen aus zwei übereinander angeordneten, an den Rändern fluiddicht miteinander verbundenen Kunststofffolien, die im Bereich des Lateral-/Ristabschnittes mindestens eine in Längsrichtung des Fluidpolsterkissens verlaufende Verbindungsnaht aufweisen, wodurch das Fluidpolsterkissen in diesem Bereich in mindestens zwei voneinander seitlich getrennte, in Längsrichtung des Pronationsgurtes jedoch durchgängige Teilkammern unterteilt ist. Hierdurch kann die Richtung der dynamischen Wellenbewegung des Fluids beeinflusst werden, da das Fluid aufgrund der Verbindungsnähte nicht mehr ungehindert über die gesamte Fluidpolsterkissenbreite in Querrichtung, sondern vor allem in Längsrichtung des Fluidpolsterkissens strömt.

Die Erfindung wird nachfolgend anhand der Zeichnungen beispielshaft näher erläutert. Es zeigen:
- Figur 1:: eine übliche Sprunggelenkschiene in einem auseinandergeklappten Zustand und in einem am Unterschenkel angelegten Zustand,
- Figur 2:: eine Darstellung einer üblichen Sprunggelenkschiene in Alleinstellung in einem Zustand, wie sie üblicherweise am Unterschenkel anliegt,

- Figur 3:: eine am Fuß eines Patienten angelegte Sprunggelenkschiene mit einem erfindungsgemäßen, verkürzt dargestellten Pronationsgurt in einer Anlage-Zwischenposition,
- Figur 4:: die erfindungsgemäße Sprunggelenkschiene in angelegtem Zustand, wobei der Fuß vom Boden abgehoben ist,
- Figur 5:: eine Darstellung entsprechend Figur 4, wobei der Fuß auf dem Boden aufgesetzt wird,
- Figur 6:: eine Draufsicht auf den erfindungsgemäßen Pronationsgurt in Alleinstellung,
- Figur 7:: eine Draufsicht auf das Fluidpolsterkissen des Pronationsgurtes von Figur 6 in Alleinstellung,
- Figur 8:: zwei Seitenansichten des Fluidpolsterkissens zur Verdeutlichung des Pumpeffekts und
- Figur 9:: eine Darstellung des am Fuß angelegten Fluidpolsterkissens in Alleinstellung.

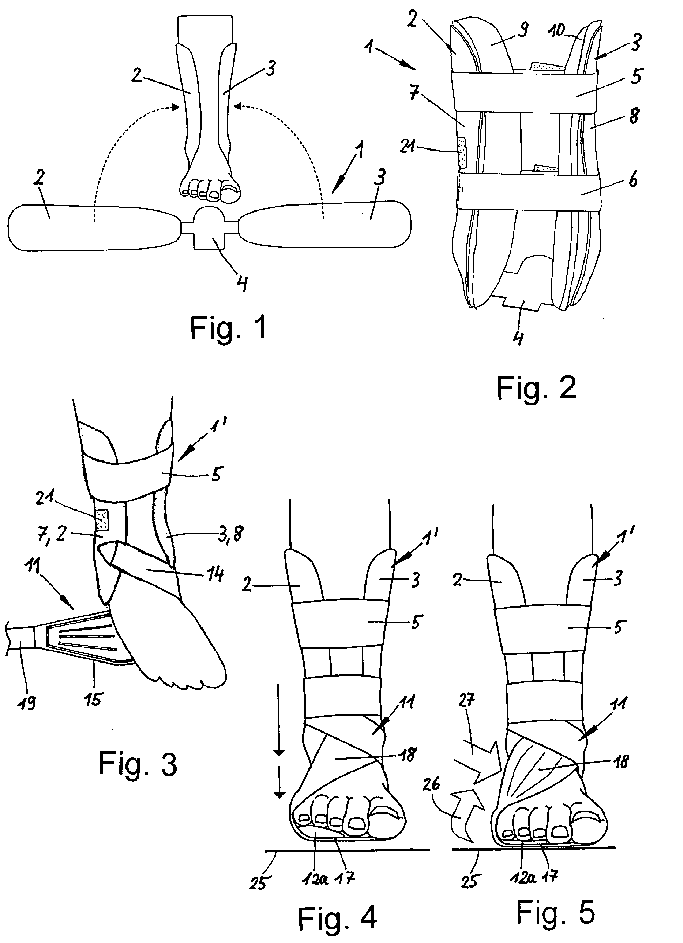

Aus den Figuren 1 und 2 ist eine übliche Sprunggelenkschiene 1 ersichtlich, die ein laterales Seitenteil 2 und ein mediales Seitenteil 3 umfasst, die miteinander an ihren unteren Enden mittels eines quer unter der Ferse eines Patienten durchführbaren Sohlenbandes 4 verbunden sind. Die Seitenteile 2, 3 können lateral und medial an den Fuß bzw. den unteren Abschnitt des Unterschenkels eines Patienten angelegt werden, wie aus Figur 1 ersichtlich, wobei sich das Sohlenband 4 unter der Fußsohle befindet und sich das laterale Seitenteil 2 von kurz oberhalb der Fußsohle über den lateralen Bereich des oberen Sprunggelenks bis in einen Seitenbereich des Unterschenkels erstreckt, während sich das mediale Seitenteil 3 von kurz oberhalb der Fußesohle über die mediale Seite des oberen Sprunggelenks bis in einen medialen Seitenbereich des Unterschenkels erstreckt.

Bei den Seitenteilen 2 und 3 handelt es sich um voneinander getrennte Teile, die lediglich im unteren Endbereich mittels des flexiblen, jedoch zugfesten Sohlenbandes 3 miteinander verbunden sind. Aufgrund dieser Ausgestaltung, die grundsätzlich bereits bekannt ist, werden derartige Schienen auch U-Schienen genannt. Die Seitenteile 2, 3 können daher zum Anlegen der Sprunggelenkschiene auseinander geschwenkt werden, so dass der Fuß dazwischen eingeführt werden kann und die Seitenteile 2, 3 auf bequeme Weise anschließend lateral bzw. medial angelegt werden können, wie aus Figur 1 ersichtlich.

Im angelegten Zustand werden die Seitenteile 2, 3 mittels eines oberen Schalengurtes 5, der in bekannter Weise zirkulär um beide Seiten herumgeschlungen wird, am Bein des Patienten gehalten. Der obere Schalengurt 4 befindet sich zweckmäßiger Weise im oberen Endbereich der beiden Seitenteile 2, 3 und ist mit einem Ende fest mit einem der beiden Seitenteile, vorzugsweise mit dem lateralen Seitenteil, verbunden, während das andere Ende mittels eines Klettverschlusses befestigt werden kann.

Wie aus Figur 2 ersichtlich, kann bei üblichen Sprunggelenkschienen 1 unterhalb des oberen Schalengurtes 5 ein unterer Schalengurt 6 in gleicher Weise wie der obere Schalengurt 5 zirkulär um beide Seitenteile 2, 3 herumgeschlungen werden, um die beiden Seitenteile 2, 3 kurz oberhalb des Sprunggelenks aneinander zu ziehen und mit der gewünschten Festigkeit am Bein des Patienten zu halten.

Die Seitenteile 2, 3 weisen üblicherweise Schalenteile 7, 8 auf, die aus Kunststoff bestehen und relativ steif ausgebildet sind, so dass sie das Sprunggelenk entsprechend stützen können. Die Wölbung der Schalenteile 7, 8 ist an die Form des Beins angepasst, um maximalen Tragekomfort zu erreichen und Druckstellen zu vermeiden. Weiterhin sind die Schalenteile 7, 8 in üblicher Weise auf der am Bein anliegenden Seite mittels Polsterungen 9, 10 ausgekleidet, die insbesondere aus einer Luftkammerpolsterung bestehen können.

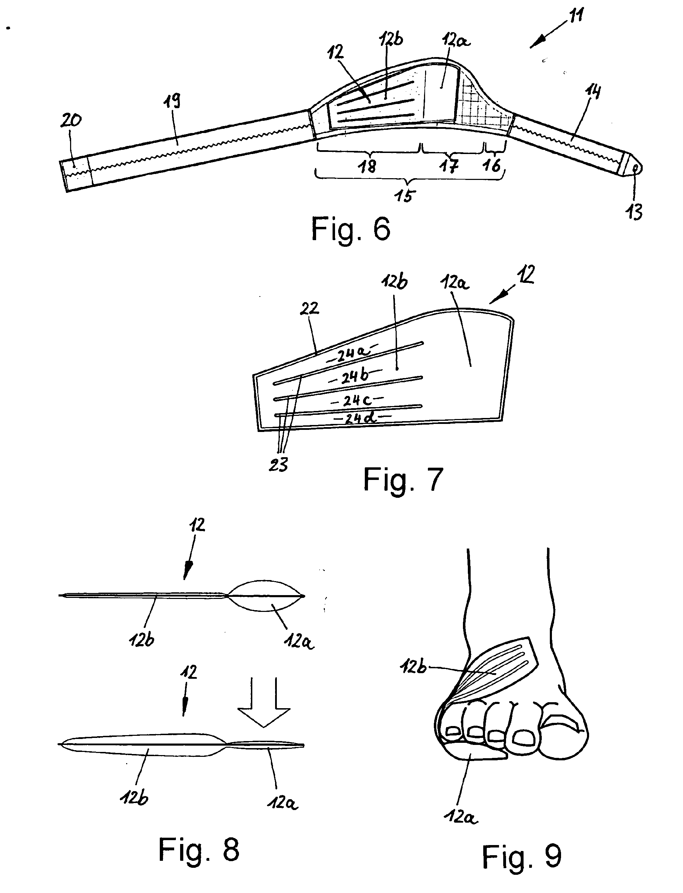

Im Gegensatz zur bekannten, in Figur 2 dargestellten Sprunggelenkschiene 1 weist die erfindungsgemäße Sprunggelenkschiene 1', die in den Figuren 3 bis 5 dargestellt ist, keinen unteren Schalengurt 6 auf, der lediglich zirkulär um die beiden Seitenteile 2, 3 herumgeschlungen ist, sondern einen speziellen Pronationsgurt 11, der in Figur 6 in Einzeldarstellung gezeigt ist. Pronationsgurte dienen bekanntermaßen dazu, den Vorfuß eines Patienten anzuheben, um insbesondere die Außenbänder zu entlasten. Im Gegensatz zu bekannten Pronationsgurten weist der erfindungsgemäße Pronationsgurt 11 jedoch ein spezielles Fluidpolsterkissen 12 auf, das beim Gehen einen dynamischen Pumpeffekt auf die anliegenden Fußbereiche ausübt und damit als Massagekissen wirkt.

Wie aus den Figur 3 bis 6 ersichtlich, weist der Pronationsgurt 11 an einem Ende ein Gurtschloss mit einem Befestigungselement 13 auf, mit dem der Pronationsgurt 11 in der unteren Hälfte des lateralen Seitenteils 2 lösbar befestigt werden kann. Bei diesem Befestigungselement 13 kann es sich beispielsweise um ein T-förmiges, seitlich über den Pronationsgurt 11 vorstehendes Ankerelement handeln, das durch eine längliche Öffnung im lateralen Schalenteil 7 hindurch eingeführt werden kann, so dass es nach einer Schwenkbewegung des Pronationsgurtes 11 das Schalenteil 7 von innen hintergreift. Andere Befestigungsmöglichkeiten sind jedoch ebenfalls denkbar, beispielsweise Klettverbindungen. Weiterhin ist es möglich, den Pronationsgurt 11 fest am lateralen Schalenteil 7 zu befestigen.

An das Gurtschloss schließt sich ein zugfester Gurtabschnitt 14. Dieser Gurtabschnitt 14 wird, wie aus den Figuren 3 bis 5 ersichtlich, diagonal über den Fußrücken nach unten bis in den medialen Seitenbereich des Mittelfußes geführt.

An den zugfesten Gurtabschnitt 14 schließt sich, wie aus Figur 6 ersichtlich, ein Gurtabschnitt 15 an, der aus einem Medialabschnitt 16, einem Sohlenabschnitt 17 und einem Lateral-/Ristabschnitt 18 besteht. Im angelegten Zustand des Pronationsgurtes 11 liegt der Medialabschnitt 16 an der medialen Seite des Fußes an, während sich der Sohlenabschnitt 17 unterhalb der Fußsohle befindet. Der Lateral-/Ristabschnitt 18 erstreckt sich von der lateralen Außenseite des Mittelfußes nach oben bis zum oberen Ende des Fußrückens. Insbesondere im Bereich des Sohlenabschnittes 17 und Lateral-/Ristabschnittes 18 ist der Pronationsgurt 11 verbreitert, wobei er im dargestellten Ausführungsbeispiel in demjenigen Bereich des Sohlenabschnittes 17, der an den Lateral-/Ristabschnitt 18 angrenzt, seine größte Breite hat. Zumindest Teilbereiche des Gurtabschnittes 15 sind vorzugsweise elastisch ausgebildet.

An den Gurtabschnitt 15 schließt sich ein zugfester Gurtabschnitt 19 an, der in gleicher Weise wie der Gurtabschnitt 14 eine gleichbleibende Breite aufweist. Beim Anlegen des Pronationsgurtes 11 wird der Gurtabschnitt 19 aus der in Figur 3 dargestellten Position zunächst unter dem Gurtabschnitt 14 hindurchgeführt und anschließend medial um das mediale Seitenteil 3 nach dorsal und von dort um das laterale Seitenteil 2 herum wieder bis in den Bereich des medialen Seitenteils 3 geführt, um dort mit seinem Ende mittels eines Klettverschlusses 20 (Figur 6) an der bereits vorhandenen Lage des Gurtabschnitts 19 festgeklettet zu werden. Aus Figur 3 ist weiterhin ein am lateralen Seitenteil 2 befestigtes Klettelement 21 ersichtlich, über das der Gurtabschnitt 19 geführt wird, so dass es auch dort zusätzlich festgeklettet wird.

Wie insbesondere aus Figur 6 ersichtlich, ist das Fluidpolsterkissen 12 im Bereich des Sohlenabschnittes 17 und des Lateral-/Ristabschnittes 18 des Pronationsgurtes 11 angeordnet. Das Fluidpolsterkissen 12 ist zweckmäßigerweise zwischen elastischen Textillagen des Gurtabschnittes 15 eingebettet, wodurch es verrutschfest im Pronationsgurt 11 festgelegt und zusätzlich vor Beschädigungen geschützt ist. Das Fluidpolsterkissen 12 ist, wie aus Figur 6 erkennbar, an die Außenkontur des Sohlenabschnittes 17 und Lateral-/Ristabschnittes 18 angepasst und erstreckt sich über die laterale Hälfe des Sohlenabschnittes 17 sowie bis in die Nähe des gegenüberliegenden Endes des Lateral-/Ristabschnittes 18. Das Fluidpolsterkissen 12 besteht somit aus einem Sohlenkissenabschnitt 12a und einem Lateral-/Ristkissenabschnitt 12b, der unmittelbar an den Sohlenkissenabschnitt 12a anschließt und einteilig mit diesem ausgebildet ist. Die Breite des Fluidpolsterkissens 12 ist nur geringfügig kleiner als diejenige der Abschnitte 17, 18.

Das Fluidpolsterkissen 12 besteht aus zwei übereinander angeordneten, flexiblen, an den Rändern fluiddicht miteinander verschweißten oder verklebten Kunststofffolien, die eine Fluidkammer umschließen. Innerhalb der Fluidkammer befindet sich eine vorbestimmte Menge Fluid, wobei der Fluidmenge derart bemessen ist, dass die Kunststofffolien etwas auseinandergedrückt werden. Wie weiterhin insbesondere aus Figur 7 ersichtlich, weist der Lateral-/Ristkissenabschnitt 12b Verbindungsnähte 23 auf, mit denen die Kunststofffolien miteinander verschweißt sind. Die Verbindungsnähte 23 verlaufen in Längsrichtung des Fluidpolsterkissen 12 bis in die Nähe, jedoch nicht ganz bis zu demjenigen Ende des Fluidpolsterkissens 12, das zum Gurtabschnitt 19 benachbart ist. Durch die drei nebeneinander angeordneten Verbindungsnähte 23 wird im vorliegenden Ausführungsbeispiel die Fluidkammer in vier längliche, nebeneinander angeordnete, jedoch voneinander seitlich durch die Verbindungsnähte 23 getrennte Teilkammern 24a, 24b, 24c, 24d unterteilt. In ihren beiden Endbereichen sind diese Teilkammern jedoch wieder miteinander in Fluidverbindung. Im Bereich des Sohlenkissenabschnittes 12a weist das Fluidpolsterkissen 12 dagegen keine Verbindungsnähte 23 auf.

Aufgrund der beschriebenen Ausbildung und Anordnung des Fluidpolsterkissens 12 wird dann, wenn der Fuß auf den Boden 25 aufgesetzt wird, der sich unterhalb der Fußsohle befindende Sohlenkissenabschnitt 12a des Fluidpolsterkissens 12 zusammengedrückt, wodurch das Fluid aus diesem Abschnitt herausgedrückt und in den angrenzenden Lateral-/Ristkissenabschnitt 12b hineingepumpt wird. Dieser Vorgang ist durch den Pfeil 26 in Figur 5 sowie durch die untere Darstellung in Figur 8 veranschaulicht. Hierdurch erweitert bzw. verdickt sich der Lateral-/Ristkissenabschnitt 12b, wodurch auf den anliegenden Teil des Fußes eine erhöhte Druckkraft aufgebracht wird, wie durch den Pfeil 27 in Figur 5 veranschaulicht. Wird der Fuß wieder vom Boden 25 hochgehoben, strömt das Fluid aufgrund bestehender Druckdifferenzen innerhalb des Fluidpolsterkissens 12 wieder nach unten in den Sohlenkissenabschnitt 12a, wodurch der Druck auf die laterale Seite und den Fußrücken wieder nachlässt. Beim Gehen wird somit eine dynamische, sich laufend ändernde Druckkraft auf den lateralen Seitenbereich des Fußes bis hinauf zum oberen Ende des Fußrückens aufgebracht, so dass das Fluidpolsterkissen 12 in diesem gesamten Fußbereich als Massagekissen wirken kann.

## Patentansprüche

1. Sprunggelenkschiene mit das Sprunggelenk eines Patienten stützenden Seitenteilen (2, 3), die auf der medialen und lateralen Seite des Unterschenkels anlegbar und an diesem befestigbar sind, und mit einem um den Fuß herumführbaren Pronationsgurt (11) zur Entlastung der Außenbänder des Sprunkgelenkes durch Anheben des Vorfußes, wobei der Pronationsgurt (11) einen quer unter der Fußsohle entlangführbaren Sohlenabschnitt (17) und einen daran anschließenden, über die laterale Außenseite des Fußes und den Fußrücken führbaren Lateral-/Ristabschnitt (18) aufweist, **dadurch gekennzeichnet, dass** der Sohlenabschnitt (17) und Lateral-/Ristabschnitt (18) des Pronationsgurtes (11) ein in beiden Abschnitten angeordnetes, als durchgehendes Massagekissen ausgebildetes Fluidpolsterkissen (12) mit Fluidfüllung aufweist, derart, dass zumindest ein Teil des im Fluidpolsterkissen (12) enthaltenen Fluids bei Komprimierung des Sohlenabschnittes (17) in den Lateral-/Ristabschnitt (18) gepumpt und bei Entlastung des Sohlenabschnittes (17) und Komprimierung des Lateral-/Ristabschnittes (18) in den Sohlenabschnitt (17) gepumpt wird, wodurch das Fluidpolsterkissen (12) im Bereich der Fußsohle, der lateralen Fußaußenseite und des Fußrückens eine dynamische Dickenänderung erfährt.

2. Sprunggelenkschiene nach Anspruch 1, **dadurch gekennzeichnet dass** sich das Fluidpolsterkissen (12) über die laterale Sohlenhälfte des Mittelfußes und von dort bis zum oberen Ende des Fußrückens erstreckt.

3. Sprunggelenkschiene nach Anspruch 1 oder 2, **dadurch gekennzeichnet dass** das Fluidpolsterkissen (12) aus zwei übereinander angeordneten, an den Rändern (22) fluiddicht miteinander verbundenen Kunststofffolien besteht, die im Bereich des Lateral-/Ristabschnittes (18) mindestens eine in Längsrichtung des Fluidpolsterkissens (12) verlaufende Verbindungsnaht (23) aufweisen, wodurch das Fluidpolsterkissen (12) in diesem Bereich in mindestens zwei voneinander seitlich getrennte, in Längsrichtung des Pronationsgurtes (11) jedoch durchgängige Teilkammern (24a, 24b, 24c, 24d) unterteilt ist.
